(19) Europäisches Patentamt / European Patent Office / Office européen des brevets

(11) **EP 1 589 947 B2**

(12) ## NEW EUROPEAN PATENT SPECIFICATION
After opposition procedure

(45) Date of publication and mention
of the opposition decision:
**30.01.2019 Bulletin 2019/05**

(45) Mention of the grant of the patent:
**15.06.2016 Bulletin 2016/24**

(21) Application number: **03800395.0**

(22) Date of filing: **31.12.2003**

(51) Int Cl.:
**A61K 9/16** (2006.01)

(86) International application number:
**PCT/US2003/041703**

(87) International publication number:
**WO 2004/060351 (22.07.2004 Gazette 2004/30)**

(54) **PHARMACEUTICAL FORMULATION WITH AN INSOLUBLE ACTIVE AGENT FOR PULMONARY ADMINISTRATION**

PHARMAZEUTISCHE FORMULIERUNG ENTHALTEND EINEN UNLÖSLICHEN WIRKSTOFF ZUR PULMONALEN VERABREICHUNG

FORMULATION PHARMACEUTIQUE AYANT UN AGENT ACTIF INSOLUBLE

(84) Designated Contracting States:
**AT BE BG CH CY CZ DE DK EE ES FI FR GB GR HU IE IT LI LU MC NL PT RO SE SI SK TR**
Designated Extension States:
**AL LT LV MK**

(30) Priority: **31.12.2002 US 437210 P**

(43) Date of publication of application:
**02.11.2005 Bulletin 2005/44**

(73) Proprietor: **Novartis AG**
**4002 Basel (CH)**

(72) Inventors:
• **TARARA, Thomas, E.**
**Burlingame, CA 94010 (US)**
• **WEERS, Jeffry, G.**
**Belmont, CA 94002 (US)**

(74) Representative: **Leon, Susanna Iris**
**Novartis Pharma AG**
**Patent Department**
**4002 Basel (CH)**

(56) References cited:
WO-A-99/16419     WO-A-02/067902
WO-A1-00/01365     US-A1- 2002 017 295
US-A1- 2002 037 316     US-A1- 2002 177 562

• **INNOVATIONS IN PHARMACEUTICAL TECHNOLOGY, vol. 1, no. 7, 2000, pages 111-116, XP001182817**
• **KIM J-C ET AL: "PREPARATION BY SPRAY DRYING OF AMPHOTERICIN B-PHOSPHOLIPID COMPOSITE PARTICLES AND THEIR ANTICELLULAR ACTIVITY" DRUG DELIVERY, ACADEMIC PRESS, ORLANDO, FL, US, vol. 8, no. 3, 2001, pages 143-147, XP001105573 ISSN: 1071-7544**
• **WIEDMANN T.S. ET AL: 'DRUG SOLUBILIZATION IN LUNG SURFACTANT' JOURNAL OF CONTROLLED RELEASE vol. 65, no. 1-2, 01 March 2000, ELSEVIER, AMSTERDAM, NL, pages 43 - 47, XP004190309**

EP 1 589 947 B2

**Description**

BACKGROUND

This invention generally relates to pharmaceutical compositions containing insoluble active agents. In particular, the insoluble active agents are provided in crystalline form and are incorporated into a matrix material. The matrix material is selected to provide desirable product characteristics for the intended application, such as aerosol dispersibility for inhalation applications or aqueous dispersibility for oral or injectable drug delivery applications.

Drug delivery technology is continuing to create new and/or better opportunities for the administration of previously difficult to administer drugs. For example, pharmaceutical formulations may be engineered to be biocompatible, to stabilize an active agent physically, to stabilize an active agent chemically, to effectively target payload, to avoid clearance, and/or to control dissolution of the active agent. One particular challenge is to formulate insoluble active agents, such as active agents having a solubility less than 1 mg/ml. Previous attempts at enhancing the in vivo performance of drug delivery systems comprising insoluble drugs include size reduction of drug crystals by micronization, milling, high pressure homogenization, and ultrasound or the development of drug delivery vehicles to solubilize or complex the poorly soluble drug, such as by using emulsions, microemulsions, solid lipid nanoparticles, and cyclodextrins.

Drug crystals have long been used as the particulates for pulmonary delivery, both for standard metered dose inhalers and for dry powder inhalers. Significant interparticle interactions exist for the micron-sized crystals currently used in aerosol delivery, necessitating that the crystals be blended with large lactose carrier particles to improve powder flow and dispersibility. However, blended pharmaceutical formulations present significant limitations for pulmonary delivery, including: (a) poor delivery efficiency (5 - 30% of the nominal dose), and (b) a dramatic dependence of lung deposition with peak inspiratory flow rate.

These deficiencies can be dramatically improved by specifically engineering the particles for pulmonary delivery. One particle engineering strategy that has been explored is the use of spray drying to create particles for inhalation. Particle engineering via spray drying affords control over attributes such as particle morphology, surface roughness, surface energy, particle density, parameters that can dramatically influence inhalation performance, but which cannot be achieved by standard crystal milling technologies. Spray dried particles have shown dramatic improvements in pulmonary delivery efficiency and reduced flow rate dependence when delivered from passive dry powder inhalers.

Presently, most spray drying strategies involve the use of aqueous-based feedstocks in which the drug substance is dissolved in the continuous water phase. One strategy is to form an amorphous glass of the drug substance. For such systems, as the solubility of the drug substance decreases below about 1 mg/ml, the active loading and allowable feedstock concentrations decrease dramatically, making spray drying by the solution-based methods impractical due to low throughput and dose limitations. This is especially true for drug substances with a low glass transition temperature, Tg.

The Fox equation relates the glass transition temperature for mixtures of glass forming agents. For active agents with a low inherent Tg, the Tg can be raised by incorporation of excipients (e.g., carbohydrates, amino acids, salts) with high Tg. The Tg of the amorphous mix, $Tg^*$, can be estimated using the Fox equation (Fox TG: Bull Am Phys Soc 1956, 1:123):

$$1/Tg^* = \frac{X_a}{Tg_a} + \frac{X_b}{Tg_b} + \cdots \frac{X_i}{Tg_i} \qquad (1)$$

Here $X_i$ represents the mass fraction of component i in the amorphous matrix, and $Tg_i$ represents the glass transition temperature for component i in the multicomponent matrix.

In pharmaceutical development it is advantageous from the standpoint of achieving good chemical and physical stability, that the glass transition temperature be ca., 50 °C higher than the storage temperature, $T_s$. Standard pharmaceutical testing includes stability testing at storage conditions of 40°C/75% relative humidity (RH). Hence $Tg^*$ should preferably be 90°C (363 °K) or greater. The Tg values of some standard glass stabilizing excipients are:

| Excipient | Tg (°K) |
|---|---|
| Sodium citrate | 443 |
| Leucine | 413 |
| Trehalose | 373 |
| Trileucine | 373 |

The dilemma facing formulators is that when the active agent has a low Tg, the amount of stabilizing excipient required may be large. This effectively limits the dose of active agent that can be practically delivered.

Using the Fox equation one can estimate the mass fraction of these excipients required to raise the Tg of an active agent with a low Tg to make a pharmaceutical product with acceptable chemical and physical stability. Assuming that $Tg\,^*$ is 363 °K, and $Tg_{API} = 283$ °C, the mass fraction of trehalose or trileucine required to achieve $Tg\,^*$ is 0.99. For leucine and sodium citrate, the mass fraction of excipient would be 0.70 and 0.61, respectively. Hence, excipients would be required to make up a large percentage of the dose, further limiting the dose that can be delivered. Hence, a need exists for the efficient and reproducible pulmonary delivery of drugs that have water solubilities less than 1 mg/ml.

Therefore, it is desirable to be able to produce a pharmaceutical formulation comprising an insoluble active agent. It is further desirable to produce the pharmaceutical formulation comprising an insoluble active agent in an economic, reproducible, and highly loaded manner. It is further desirable to produce an inhaleable pharmaceutical formulation comprising an insoluble active agent that may be effectively aerosolized and delivered to the lungs of a user.

## SUMMARY

The present invention satisfies these needs. In one aspect of the invention, a pharmaceutical formulation comprises an insoluble active agent in a matrix comprising hydrophobic material.

In another aspect of the invention, a pharmaceutical formulation for pulmonary administration comprises particulates comprising an active agent particle in a lipid matrix, the active agent having a solubility in water of less than 1.0 mg/ml; wherein at least 90% of the active agent particles in the pharmaceutical formulation have a geometric diameter less than 3 $\mu$m and wherein the particulates have a mass median diameter less than 20 $\mu$m.

In another aspect of the invention, a method of making a pharmaceutical formulation for pulmonary administration comprises suspending active agent particles and a hydrophobic material in a liquid feedstock, wherein at least 90% of the active agent particles have a geometric diameter less than 3 $\mu$m; and spray drying the feedstock suspension to produce particulates comprising an active agent particle at least partially in the hydrophobic material.

In another aspect of the invention, a pharmaceutical formulation is prepared by suspending active agent particles and a hydrophobic material in a liquid feedstock, wherein at least 90% of the active agent particles have a geometric diameter less than 3 $\mu$m; and spray drying the feedstock suspension to produce particulates comprising an active agent particle at least partially in the hydrophobic material.

In another aspect of the invention, a pharmaceutical formulation for pulmonary administration comprises particulates comprising an amphotericin B particle in a lipid matrix; wherein at least 90% of the amphotericin B particles in the pharmaceutical formulation have a geometric diameter less than 3 $\mu$m and wherein the particulates have a mass median diameter less than 20 $\mu$m.

In another aspect of the invention, a pharmaceutical formulation for pulmonary administration comprises particulates comprising an amphotericin B particle in a lipid matrix; wherein the particulates are hollow and/or porous and wherein the particulates have a mass median diameter less than 20 $\mu$m.

In another aspect of the invention, a pharmaceutical formulation for pulmonary administration comprises particulates comprising an amphotericin B particle in a lipid matrix; wherein the particulates have a bulk density less than 0.5 g/cm$^3$ and wherein the particulates have a mass median diameter less than 20 $\mu$m.

In another aspect of the invention, a method of making a pharmaceutical formulation for pulmonary administration comprises suspending amphotericin B particles and a hydrophobic material in a liquid feedstock, wherein at least 90% of the active agent particles have a geometric diameter less than 3 $\mu$m; and spray drying the feedstock suspension to produce particulates comprising amphotericin B at least partially in the hydrophobic material.

In another aspect of the invention, a pharmaceutical formulation is prepared by suspending amphotericin B particles and a hydrophobic material in a liquid feedstock, wherein at least 90% of the active agent particles have a geometric diameter less than 3 $\mu$m; and spray drying the feedstock suspension to produce particulates comprising amphotericin B at least partially in the hydrophobic material.

In another aspect of the invention, a method of making a pharmaceutical formulation for pulmonary administration comprises suspending amphotericin B particles in a liquid feedstock, the liquid feedstock having a lipid and a blowing agent dissolved or suspended therein; and spray drying the feedstock suspension to produce hollow and/or porous particulates comprising amphotericin B and the lipid.

In another aspect of the invention, a pharmaceutical formulation is prepared by suspending amphotericin B particles in a liquid feedstock, the liquid feedstock having a lipid and a blowing agent dissolved or suspended therein; and spray drying the feedstock suspension to produce hollow and/or porous particulates comprising amphotericin B and the lipid.

DRAWINGS

These features, aspects, and advantages of the present invention will become better understood with regard to the following description, appended claims, and accompanying drawings which illustrate exemplary features of the invention. However, it is to be understood that each of the features can be used in the invention in general, not merely in the context of the particular drawings, and the invention includes any combination of these features, where:

Fig. 1 depicts a plot of the characteristic time for Ostwald ripening as a function of particle diameter and active agent solubility in water;

Figure 2 depicts a plot of flow rate dependence of deposition in an Anderson Cascade Impactor (ACI) for an Amphotericin B powder;

Figure 3 depicts a plot of stability of amphotericin B powder emitted dose efficiency using the Turbospin DPI device at 60 L min$^{-1}$;

Figure 4 depicts a plot of stability of amphotericin B powder aerosol performance using the Turbospin DPI device at 28.3 L min$^{-1}$;

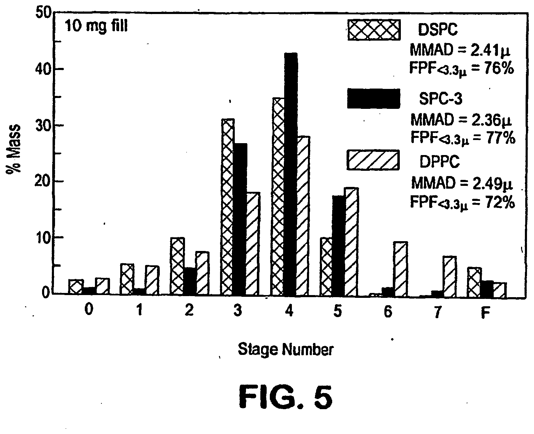

Figure 5 depicts a plot of aerosol performance of amphotericin B powders comprised of various phosphatidylcholines;

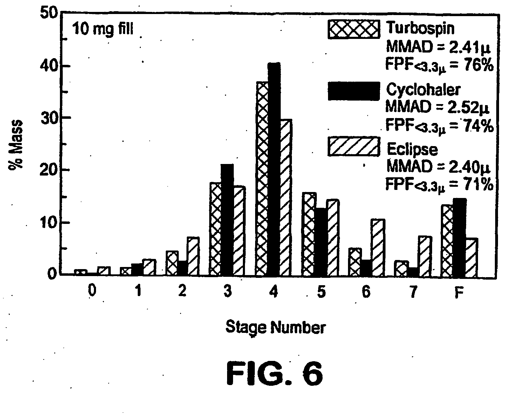

Figure 6 depicts a plot of aerosol performance of 70% amphotericin B powder using various passive DPI devices at 56.6 L min$^{-1}$; and

Figures 7A through 7E are schematic sectional side views showing the operation of a dry powder inhaler that may be used to aerosolize a pharmaceutical formulation according to the invention.

DEFINITIONS

"Active agent" as described herein includes an agent, drug, compound, composition of matter or mixture thereof which provides some diagnostic, prophylactic, or pharmacologic, often beneficial, effect. This includes foods, food supplements, nutrients, drugs, vaccines, vitamins, and other beneficial agents. As used herein, the terms further include any physiologically or pharmacologically active substance that produces a localized or systemic effect in a patient. The active agent that can be delivered includes antibiotics, antibodies, antiviral agents, anepileptics, analgesics, anti-inflammatory agents and bronchodilators, and viruses and may be inorganic and organic compounds, including, without limitation, drugs which act on the peripheral nerves, adrenergic receptors, cholinergic receptors, the skeletal muscles, the cardiovascular system, smooth muscles, the blood circulatory system, synaptic sites, neuroeffector junctional sites, endocrine and hormone systems, the immunological system, the reproductive system, the skeletal system, autacoid systems, the alimentary and excretory systems, the histamine system and the central nervous system. Suitable agents may be selected from, for example, polysaccharides, steroids, hypnotics and sedatives, psychic energizers, tranquilizers, anticonvulsants, muscle relaxants, antiinfectives, anti-migraine agents, antiparkinson agents, analgesics, anti-inflammatories, muscle contractants, antimicrobials, antimalarials, hormonal agents including contraceptives, sympathomimetics, polypeptides, and proteins capable of eliciting physiological effects, diuretics, lipid regulating agents, antiandrogenic agents, antiparasitics, neoplastics, antineoplastics, hypoglycemics, nutritional agents and supplements, growth supplements, fats, antienteritis agents, electrolytes, vaccines and diagnostic agents.
Examples of active agents useful in this invention include but are not limited to analgesics/antirheumatics such as morphine, codeine, fentanyl, indomethacin, naproxen, and piroxicam; antiallergics such as pheniramine, dimethindene, terfenadine, loratidine, and doxylamine; antibiotics such as rifampicin, ethambutol, and thiacetazone; antiepileptics such as carbamazepine, clonazepam, alprazolam, medazolam, mesuximide, phenytoin, and valproic acid; antimycotics such as natamycin, amphotericin B, voriconazole, and miconazole; corticoids such as aldosterone, dexamethasone, triamcinolone, budesonide, fluticasone, and beclomethoasone; migraine agents such as lisuride, methysergide, dihydroergotamine, ergotamine; psychotropics such as benzodiazepines and clormethiazole; anticancer agents such as mephalan, carmustine, lomustine, cyclophosphamide, ifosamide, trofosamide, chlorambucil, fluorouracil, methotrexate, vinblastine, vincristine, dactinomycin, and camptothecins; cytostatic drugs such as Ara-C, FudR, and 5FU; Virostatic drugs such as AZT, ddC, and ddI; MRI contrast agents such as GdDTPA; and asthma agents such as nonsteroidal inflammatory agents such as VLA-4 inhibitors and phosphodiesterase inhibitors (e.g., PD-4 inhibitors).
Additional active agents include, but are not limited to cyclosporine, ciprofloxacin, amikacin, tobramycin, pentamidine isethionate, beclomethasone dipropionate, triamcinolone acetamide, flunisolide, fluticasone, fluticasone propionate, salmeterol xinofoate, formeterol fumarate, ergotamine tartrate, doxorubicin, mitoxantrone, progesterone, micronazole, piroxicam, tacrolimus, sirolimus, indomethacin, econazole, paramethoxy cinnamate, THC, nicotine, halofantrine, statins, taxol, taxotere, alfaxlone, erythromycin, albendazole, nitroscanate, dantrolene, caphalone, tilmicosine, nitazoxanide, fluoroquinolone (e.g., ciprofloxacin), tilmicosin, all-trans retinoic acid and the analogues, agonists and antagonists of the above. The active agents may be in various forms, such as insoluble charged or uncharged molecules, components of molecular complexes or pharmacologically acceptable salts. The active agents may be naturally occurring molecules or they may be recombinantly produced, or they may be analogs of the naturally occurring or recombinantly produced active agents with one or more amino acids added or deleted. Further, the active agent may comprise live attenuated or killed viruses suitable for use as vaccines.

As used herein, the term "emitted dose" or "ED" refers to an indication of the delivery of dry powder from a suitable inhaler device after a firing or dispersion event from a powder unit or reservoir. ED is defined as the ratio of the dose delivered by an inhaler device (described in detail below) to the nominal dose (*i.e.*, the mass of powder per unit dose placed into a suitable inhaler device prior to firing). The ED is an experimentally-determined amount, and is typically determined using an *in-vitro* device set up which mimics patient dosing. To determine an ED value, a nominal dose of dry powder (as defined above) is placed into a suitable dry powder inhaler, which is then actuated, dispersing the powder. The resulting aerosol cloud is then drawn by vacuum from the device, where it is captured on a tared filter attached to the device mouthpiece. The amount of powder that reaches the filter constitutes the delivered dose. For example, for a 5 mg, dry powder-containing blister pack placed into an inhalation device, if dispersion of the powder results in the recovery of 4 mg of powder on a tared filter as described above, then the ED for the dry powder composition is: 4 mg (delivered dose)/5 mg (nominal dose) x 100 = 80%.

As used herein, the term "insoluble" refers to a water solubility of less than 1.0 mg/ml measure at about physiologically neutral pH of about 5.0 - 8.0.

"Mass median diameter" or "MMD" is a measure of median particle size, since the powders of the invention are generally polydisperse (i.e., consist of a range of particle sizes). MMD values as reported herein are determined by centrifugal sedimentation and/or by laser defraction, although any number of commonly employed techniques can be used for measuring mean particle size.

"Mass median diameter" or "MMAD" is a measure of mean particle size, since the powders of the invention are generally polydisperse (i.e., consist of a range of particle sizes). "Mass median aerodynamic diameter" or "MMAD" is a measure of the aerodynamic size of a dispersed particle. The aerodynamic diameter is used to describe an aerosolized powder in terms of its settling behavior, and is the diameter of a unit density sphere having the same settling velocity, generally in air, as the particle. The aerodynamic diameter encompasses particle shape, density and physical size of a particle. As used herein, MMAD refers to the midpoint or median of the aerodynamic particle size distribution of an aerosolized powder determined by cascade impaction.

As used herein, "passive dry powder inhaler" refers to an inhalation device which relies upon the patient's inspiratory effort to disperse and aerosolize a drug formulation contained within the device and does not include inhaler devices which comprise a means for providing energy to disperse and aerosolize the drug formulation, such as pressurized gas and vibrating or rotating elements.

<u>DESCRIPTION</u>

The present invention relates to a pharmaceutical formulation which comprises an insoluble active agent, the production of the pharmaceutical formulation, and the use of the pharmaceutical formulation. Although the process is illustrated in the context of formulating an aerosolizable pharmaceutical formulation, the present invention can be used in other processes and should not be limited to the examples provided herein.

The pharmaceutical formulations of the present invention provide an insoluble active agent with improved or enhanced bioavailability, delivery efficiency, chemical stability, physical stability, and/or producibility. In one version, the pharmaceutical formulation comprises an insoluble active agent, in crystalline form, at least partially incorporated in a matrix material. The matrix material is selected to provide desired characteristics, such as aerosol dispersibility or improved suspension within a liquid medium. The pharmaceutical formulation of the present invention may be formed for extended release or for immediate release.

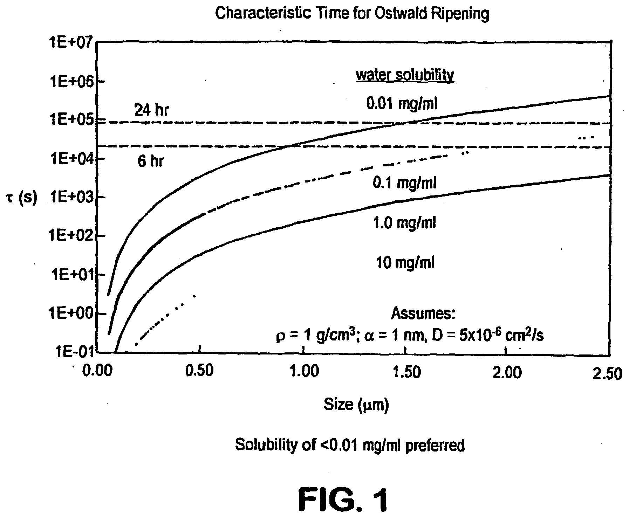

The pharmaceutical formulation comprises an active agent that is poorly water-soluble. Accordingly, in one version, the pharmaceutical formulation comprises an active agent having a solubility in water of less than 1.0 mg/ml. In a particular version, the pharmaceutical formulation comprises an active agent having a solubility in water of less than 0.1 mg/ml. Figure 1 depicts a plot of the characteristic time for Ostwald ripening as a function of particle diameter and active agent solubility in water. As can be seen from the figure, active agents having a water solubility of less than 0.1 mg/ml can achieve stability over a range of processing conditions. In another particular version, the pharmaceutical formulation comprises an active agent having a solubility in water of from 0.1 mg/ml to 1.0 mg/ml. It has been unexpectedly discovered that active agents with a water solubility of from 0.1 mg/ml to 1.0 mg/ml can be effectively formulated. This is unexpected in view of Lifshitz-Slezov-Wagner theory which predicts that active agents with a solubility in the range of 0.1 - 1.0 mg/ml would suffer from Ostwald ripening under a wide range of processing conditions generally used to produce aerosolizable pharmaceutical formulations.

In one version, the pharmaceutical formulation comprises an insoluble active agent particle that is in a matrix material. It has been discovered that it is advantageous to use small diameter insoluble active agent particles. In particular for an aerosolizable pharmaceutical formulation, it has been determined to be desirable to use insoluble active agent particles that are less than 3 $\mu$m in diameter. Accordingly, in one version, the pharmaceutical formulation of the present invention is produced using insoluble active agent particles, at least 20% of which have a diameter less than 3 $\mu$m, and more preferably at least 50% of which have a diameter less than 3 $\mu$m. In a preferred version, at least 90% of the mass of particles of active agent used to make the pharmaceutical formulation are less than 3.0 $\mu$m in diameter, more preferably at least 95% of the mass of particles of active agent used to make the pharmaceutical formulation are less than 3.0 $\mu$m in diameter. Alternatively or additionally, at least 50% of the mass of particles of active agent used to make the pharmaceutical formulation are between 0.5 $\mu$m and 3.0 $\mu$m in diameter, and more preferably between 1.0 $\mu$m and 3.0 $\mu$m. In another version, it is desirable for the insoluble active agent particles to be less than 2.5 $\mu$m, and more preferably less than 2.0 $\mu$m. Accordingly, in this version, the pharmaceutical formulation of the present invention is produced using insoluble active agent particles, most of which have a diameter less than 2.5 $\mu$m, and more preferably less than 2.0 $\mu$m. In one version, at least 90% of the mass of particles of active agent used to make the pharmaceutical formulation are less than 2.5 $\mu$m in diameter, more preferably at least 95% of the mass of particles of active agent used to make the pharmaceutical formulation are less than 2.5 $\mu$m in diameter. Alternatively or additionally, at least 50% of the mass of particles of active agent used to make the pharmaceutical formulation are between 0.5 $\mu$m and 2.5 $\mu$m in diameter, and more preferably between 1.0 $\mu$m and 2.5 $\mu$m. The insoluble active agent particle is in crystalline form.

In many instances, the insoluble active agent in bulk form has a particle size greater than 3.0 $\mu$m, and in many cases greater than 10 $\mu$m. Accordingly, in one version of the invention, the bulk insoluble active agent is subjected to a size reduction process to reduce the particle size to below 3 microns prior to incorporating the insoluble active agent particles in the matrix material. Suitable size reduction processes are known in the art and include supercritical fluid processing methods such as disclosed in WO 95/01221, WO 96/00610, and WO 98/36825, cryogenic milling, wet milling, ultrasound, high pressure homogenization, microfluidization, crystallization processes, and in processes disclosed in U.S. Patent Nos. 5,858,410, all of which are hereby incorporated in their entirety by reference. Once the desired particle size of the active agent has been achieved, the resulting insoluble active agent particles are collected and then incorporated into a matrix material.

It has been unexpectedly discovered that it is particularly advantageous for the particle size of the insoluble active agent particles to be below 3.0 $\mu$m, preferably below 2.5 $\mu$m, and most preferably below about 2.0 $\mu$m, in order to provide highly dispersible, homogenous compositions of active agent incorporated into the matrix material. It has been discovered that if the active agent particle size is greater than about 3.0 microns, a heterogeneous composition results comprising active agent incorporated in the matrix material and particles comprising active agent without any matrix material. These heterogeneous compositions often exhibit poor powder flow and dispersibility. Accordingly, a preferred embodiment is directed to homogeneous compositions of active agent incorporated in a matrix material without any unincorporated active agents particles. However, in some cases, such heterogeneous compositions may be desirable in order to provide a desired pharmacokinetic profile of the active agent to be administered, and in these cases, a large active agent particle may be used.

In one version, the insoluble active agent is incorporated in a matrix that forms a discrete particulate, and the pharmaceutical formulation comprises a plurality of the discrete particulates. The particulates may be sized so that they are effectively administered and/or so that they are highly bioavailable. For example, for an aerosolizable pharmaceutical formulation, the particulates are of a size that allows the particulates to be aerosolized and delivered to a user's respiratory tract during the user's inhalation. Accordingly, in one version, the pharmaceutical formulation comprises particulates having a mass median diameter less than 20 $\mu$m, more preferably less than 10 $\mu$m, and more preferably less than 5 $\mu$m.

The matrix material may comprise a hydrophobic or a partially hydrophobic material. For example, the matrix material may comprise a lipid, such as a phospholipid, and/or a
hydrophobic amino acids, such as leucine and tri-leucine. Examples of phospholipid matrices are described in PCT Publications WO 99/16419, WO 99/16420, WO 99/16422, WO 01/85136 and WO 01/85137 and in U.S. Patents 5,874,064; 5,855,913; 5,985,309; and 6,503,480, all of which are incorporated herein by reference in their entireties. Examples of hydrophobic amino acid matrices are described in U.S. Patents 6,372,258 and 6,358,530, both of which are incorporated herein by reference in their entireties.

The pharmaceutical formulation may be advantageously produced using a spray drying process. In one version, the insoluble active agent particles having a diameter less than 3 $\mu$m and the matrix material are added to an aqueous feedstock to for a feedstock suspension. The feedstock suspension is then spray dried to produce dried particulates comprising the matrix material and the insoluble active agent. Suitable spray drying processes are known in the art, for example as disclosed in PCT WO 99/16419 and U.S. Patent Nos. 6,077,543, 6,051,256, 6,001,336, 5,985,248, and 5,976,574, all of which are incorporated herein by reference in their entireties.

Optionally, the size reduction of the insoluble active agent particles may be achieved as part of a single process to form the final particles. A single process of this type is described in PCT WO 99/16419, which is incorporated herein by reference in its entirety. In applying this process for insoluble active agents, it is necessary to provide suitable control over the particle size of the active agent to be incorporated into the matrix material.

In one version, the phospholipid is a saturated phospholipid, such as one or more phosphatidylcholines. Preferred acyl chain lengths are 16:0 and 18:0 (i.e. palmitoyl and stearoyl). The phospholipid content may be determined by the active agent activity, the mode of delivery, and other factors. In general, the phospholipid content is in the range from about 5% to up to 99.9% w/w, preferably 20% w/w - 80% w/w. Thus, active agent loading can vary between about 0.1% and 95% w/w, preferably 20 - 80% w/w.

Phospholipids from both natural and synthetic sources are compatible with the present invention and may be used in varying concentrations to form the structural matrix. Generally compatible phospholipids comprise those that have a gel to liquid crystal phase transition greater than about 40°C. Preferably the incorporated phospholipids are relatively long chain (i.e. $C_{16}$-$C_{22}$) saturated lipids and more preferably comprise saturated phospholipids, most preferably saturated phosphatidylcholines having acyl chain lengths of 16:0 or 18:0 (palmitoyl and stearoyl). Exemplary phospholipids useful in the disclosed stabilized preparations comprise, phosphoglycerides such as dipalmitoylphosphatidylcholine, disteroylphosphatidylcholine, diarachidoylphosphatidylcholine dibehenoylphosphatidylcholine, diphosphatidyl glycerol, short-chain phosphatidylcholines, long-chain saturated phosphatidylethanolamines, long-chain saturated phosphatidylserines, long-chain saturated phosphatidylglycerols, long-chain saturated phosphatidylinositols.

When phospholipids are utilized as the matrix material, it is preferable to further incorporate a polyvalent cation into the feedstock, as disclosed in PCT WO 01/85136 and WO 01/85137, hereby incorporated in their entirety by reference. Suitable polyvalent cations are preferably a divalent cation including calcium, magnesium, zinc, iron, and the like. The polyvalent cation is present in an amount effective to increase the Tm of the phospholipid such that the particulate composition exhibits a Tm which is greater than its storage temperature Ts by at least 20 °C, preferably at least 40°C. The molar ratio of polyvalent cation to phospholipid should be at least 0.05, preferably 0.05 - 2.0, and most preferably 0.25 - 1.0. A molar ratio of polyvalent cation:phospholipid of about 0.50 is particularly preferred. Calcium is the particularly preferred polyvalent cation and is provided as calcium chloride.

In addition to the phospholipid, a co-surfactant or combinations of surfactants, including the use of one or more in the liquid phase and one or more associated with the particulate compositions are contemplated as being within the scope of the invention. By "associated with or comprise" it is meant that the particulate compositions may incorporate, adsorb, absorb, be coated with or be formed by the surfactant. Surfactants include fluorinated and nonfluorinated compounds and are selected from the group consisting of saturated and unsaturated lipids, nonionic detergents, nonionic block copolymers, ionic surfactants and combinations thereof. In those embodiments comprising stabilized dispersions, such nonfluorinated surfactants will preferably be relatively insoluble in the suspension medium. It should be emphasized that, in addition to the aforementioned surfactants, suitable fluorinated surfactants are compatible with the teachings herein and may be used to provide the desired preparations.

Compatible nonionic detergents suitable as co-surfactants comprise: sorbitan esters including sorbitan trioleate (Span™ 85), sorbitan sesquioleate, sorbitan monooleate, sorbitan monolaurate, polyoxyethylene (20) sorbitan monolaurate, and polyoxyethylene (20) sorbitan monooleate, oleyl polyoxyethylene (2) ether, stearyl polyoxyethylene (2) ether, lauryl polyoxyethylene (4) ether, glycerol esters, and sucrose esters. Other suitable nonionic detergents can be easily identified using McCutcheon's Emulsifiers and Detergents (McPublishing Co., Glen Rock, New Jersey) which is incorporated herein in its entirety. Preferred block copolymers include diblock and triblock copolymers of polyoxyethylene and polyoxypropylene, including poloxamer 188 (Pluronic™ F-68), poloxamer 407 (Pluronic™ F-127), and poloxamer 338. Ionic surfactants such as sodium sulfosuccinate, and fatty acid soaps may also be utilized.

Other lipids including glycolipids, ganglioside GM1, sphingomyelin, phosphatidic acid, cardiolipin; lipids bearing polymer chains such as polyethylene glycol, chitin, hyaluronic acid, or polyvinylpyrrolidone; lipids bearing sulfonated mono-, di-, and polysaccharides; fatty acids such as palmitic acid, stearic acid, and oleic acid; cholesterol, cholesterol esters, and cholesterol hemisuccinate may also be used in accordance with the teachings of this invention.

It will further be appreciated that the pharmaceutical formulation according to the invention may, if desired, contain a combination of two or more active ingredients. The agents may be provided in combination in a single species of particulate composition or individually in separate species of particulate compositions. For example, two or more active agents may be incorporated in a single feed stock preparation and spray dried to provide a single particulate composition species comprising a plurality of active agents. Conversely, the individual actives could be added to separate stocks and spray dried separately to provide a plurality of particulate composition species with different compositions. These individual species could be added to the suspension medium or dry powder dispensing compartment in any desired proportion and placed in the aerosol delivery system as described below. Further, the pharmaceutical formulation may be combined with one or more other active or bioactive agents to provide the desired dispersion stability or powder dispersibility.

Based on the foregoing, it will be appreciated by those skilled in the art that a wide variety of active agents may be incorporated in the disclosed particulate compositions. Accordingly, the list of preferred active agents listed herein is exemplary only and not intended to be limiting. It will also be appreciated by those skilled in the art that the proper amount of agent and the timing of the dosages may be determined for the particulate compositions in accordance with already existing information and without undue experimentation.

The pharmaceutical formulation of the present invention may also include a biocompatible, preferably biodegradable polymer, copolymer, or blend or other combination thereof. In this respect useful polymers comprise polylactides, polylactide-glycolides, cyclodextrins, polyacrylates, methylcellulose, carboxymethylcellulose, polyvinyl alcohols, polyanhydrides, polylactams, polyvinyl pyrrolidones, polysaccharides (dextrans, starches, chitin, chitosan, etc.), hyaluronic acid, proteins, (albumin, collagen, gelatin, etc.). Examples of polymeric resins that would be useful for the preparation of perforated ink microparticles include: styrene-butadiene, styrene-isoprene, styrene-acrylonitrile, ethylene-vinyl acetate, ethylene-acrylate, ethylene-acrylic acid, ethylene-methylacrylatate, ethylene-ethyl acrylate, vinyl-methyl methacrylate, acrylic acid-methyl methacrylate, and vinyl chloride-vinyl acetate. Those skilled in the art will appreciate that, by selecting the appropriate polymers, the delivery efficiency of the particulate compositions and/or the stability of the dispersions may be tailored to optimize the effectiveness of the active or agent.

Besides the aforementioned polymer materials and surfactants, it may be desirable to add other excipients to a particulate composition to improve particle rigidity, production yield, emitted dose and deposition, shelf-life and patient acceptance. Such optional excipients include, but are not limited to: coloring agents, taste masking agents, buffers, hygroscopic agents, antioxidants, and chemical stabilizers. Further, various excipients may be incorporated in, or added to, the particulate matrix to provide structure and form to the particulate compositions (i.e. microspheres such as latex particles). In this regard it will be appreciated that the rigidifying components can be removed using a post-production technique such as selective solvent extraction.

Other excipients may include, but are not limited to, carbohydrates including monosaccharides, disaccharides and polysaccharides. For example, monosaccharides such as dextrose (anhydrous and monohydrate), galactose, mannitol, D-mannose, sorbitol, sorbose and the like; disaccharides such as lactose, maltose, sucrose, trehalose, and the like; trisaccharides such as raffinose and the like; and other carbohydrates such as starches (hydroxyethylstarch), cyclodextrins and maltodextrins. Other excipients suitable for use with the present invention, including amino acids, are known in the art such as those disclosed in WO 95/31479, WO 96/32096, and WO 96/32149. Mixtures of carbohydrates and amino acids are further held to be within the scope of the present invention. The inclusion of both inorganic (e.g. sodium chloride, etc.), organic acids and their salts (e.g. carboxylic acids and their salts such as sodium citrate, sodium ascorbate, magnesium gluconate, sodium gluconate, tromethamine hydrochloride, etc.) and buffers is also contemplated. The inclusion of salts and organic solids such as ammonium carbonate, ammonium acetate, ammonium chloride or camphor are also contemplated.

Yet other version of the pharmaceutical formulation include particulate compositions that may comprise, or may be coated with, charged species that prolong residence time at the point of contact or enhance penetration through mucosae. For example, anionic charges are known to favor mucoadhesion while cationic charges may be used to associate the formed microparticulate with negatively charged bioactive agents such as genetic material. The charges may be imparted through the association or incorporation of polyanionic or polycationic materials such as polyacrylic acids, polylysine, polylactic acid and chitosan.

Whatever components are selected, the first step in particulate production typically comprises feed stock preparation. The concentration of the active agent used is dependent on the amount of agent required in the final powder and the performance of the delivery device employed (e.g., the fine particle dose for a MDI or DPI). As needed, cosurfactants such as poloxamer 188 or span 80 may be dispersed into this annex solution. Additionally, excipients such as sugars and starches can also be added.

Optionally, a polyvalent cation-containing oil-in-water emulsion may then be formed in a separate vessel. The oil employed is preferably a fluorocarbon (e.g., perfluorooctyl bromide, perfluorooctyl ethane, perfluorodecalin) which is emulsified with a phospholipid. For example, polyvalent cation and phospholipid may be homogenized in hot distilled water (e.g., 60°C) using a suitable high shear mechanical mixer (e.g., Ultra-Turrax model T-25 mixer) at 8000 rpm for 2 to 5 minutes. Typically 5 to 25 g of fluorocarbon is added dropwise to the dispersed surfactant solution while mixing. The resulting polyvalent cation-containing perfluorocarbon in water emulsion is then processed using a high pressure homogenizer to reduce the particle size. Typically the emulsion is processed at 12,000 to 18,000 psi, 5 discrete passes.

The insoluble active agent suspension and perfluorocarbon emulsion are then combined and fed into the spray dryer. Operating conditions such as inlet and outlet temperature, feed rate, atomization pressure, flow rate of the drying air, and nozzle configuration can be adjusted in accordance with the manufacturer's guidelines in order to produce the required particle size, and production yield of the resulting dry particles. Exemplary settings are as follows: an air inlet temperature between 60°C and 170°C; an air outlet between 40°C to 120°C; a feed rate between 3 ml to about 15 ml per minute; and an aspiration air flow of 300 L/min. and an atomization air flow rate between 25 to 50 L/min. The selection of appropriate apparatus and processing conditions are well within the purview of a skilled artisan in view of the teachings herein and may be accomplished without undue experimentation. In any event, the use of these and substantially equivalent methods provide for the formation of aerodynamically light microparticles with particle diameters appropriate for aerosol deposition into the lung.

The pharmaceutical formulation may be formulated to comprise particulates that may be used in the form of dry powders or in the form of stabilized dispersions comprising a non-aqueous phase. Accordingly, the dispersions or powders of the present invention may be used in conjunction with metered dose inhalers (MDIs), as described in PCT Publication WO 99/16422, with dry powder inhalers (DPIs), as described in PCT Publication WO 99/16419, nebulizers, as described in PCT Publication WO 99/16420, and/or in liquid dose instillation (LDI) techniques, as described in PCT Publication WO 99/16421, to provide for effective drug delivery.

In one version, the pharmaceutical formulation may be delivered to the lungs of a user in the form of a dry powder. Accordingly, the pharmaceutical formulation comprises a dry powder that may be effectively delivered to the deep lungs or to another target site. The pharmaceutical formulation according to this version of the invention is in the form of a dry powder which is composed of particles having a particle size selected to permit penetration into the alveoli of the lungs. Ideally for this delivery, the mass median aerodynamic diameter of the particles is less than 5 $\mu$m, and preferably less than 3 $\mu$m, and most preferably between 1 $\mu$m and 3 $\mu$m. The mass median diameter of the particles may be less than 20 $\mu$m, more preferably less than 10 $\mu$m, more preferably less than 6 $\mu$m, and most preferably from 2 $\mu$m to 4 $\mu$m. The delivered dose efficiency (DDE) of these powders may be greater than 30%, more preferably greater than 40%, more preferably greater than 50%, more preferably greater than 60%, and most preferably greater than 70%. These dry powders have a moisture content less than about 15% by weight, more preferably less than about 10% by weight, and most preferably less than about 5% by weight. Such powders are described in WO 95/24183, WO 96/32149, WO 99/16419, WO 99/16420, and WO 99/16422, all of which are all incorporated herein by reference in their entireties.

In one version, the pharmaceutical formulation comprises an insoluble active agent incorporated into a phospholipid matrix. The pharmaceutical formulation may comprise phospholipid matrices that incorporate the active agent and that are in the form of particulates that are hollow and/or porous microstructures, as described in the aforementioned in WO 99/16419, WO 99/16420, WO 99/16422, WO 01/85136 and WO 01/85137. The hollow and/or porous microstructures are particularly useful in delivering the active agent to the lungs because the density, size, and aerodynamic qualities of the hollow and/or porous microstructures are ideal for transport into the deep lungs during a user's inhalation. In addition, the phospholipid-based hollow and/or porous microstructures reduce the attraction forces between particles, making the pharmaceutical formulation easier to deagglomerate during aerosolization and improving the flow properties of the pharmaceutical formulation making it easier to process. The hollow and/or porous microstructures may exhibit, define or comprise voids, pores, defects, hollows, spaces, interstitial spaces, apertures, perforations or holes, and may be spherical, collapsed, deformed or fractured particulates.

The hollow and/or porous microstructures may be formed by spray drying, as disclosed in WO 99/16419. The spray drying process results in the formation of a pharmaceutical formulation comprising particulates having a relatively thin porous wall defining a large internal void. The spray drying process is also often advantageous over other processes in that the particles formed are less likely to rupture during processing or during deagglomeration. The preparation to be spray dried or feedstock can be any solution, course suspension, slurry, colloidal dispersion, or paste that may be atomized using the selected spray drying apparatus. For the case of insoluble active agents, the feedstock may comprise a suspension as described above. Alternatively, a dilute solution and/or one or more solvents may be utilized in the feedstock. In preferred embodiments the feed stock will comprise a colloidal system such as an emulsion, reverse emulsion, microemulsion, multiple emulsion, particulate dispersion, or slurry. Typically the feed is sprayed into a current of warm filtered air that evaporates the solvent and conveys the dried product to a collector. The spent air is then exhausted with the solvent. Commercial spray dryers manufactured by Buchi Ltd. or Niro Corp. may be modified for use to produce the pharmaceutical formulation. Examples of spray drying methods and systems suitable for making the dry powders of the present invention are disclosed in U.S. Pat. Nos. 6,077,543, 6,051,256, 6,001,336, 5,985,248, and 5,976,574, all of which are incorporated herein by reference in their entireties.

In some instances dispersion stability and dispersibility of the spray dried pharmaceutical formulation can be improved by using a blowing agent, as described in the aforementioned WO 99/16419. This process forms an emulsion, optionally stabilized by an incorporated surfactant, typically comprising submicron droplets of water immiscible blowing agent dispersed in an aqueous continuous phase. The blowing agent may be a fluorinated compound (e.g. perfluorohexane, perfluorooctyl bromide, perfluorooctyl ethane, perfluorodecalin, perfluorobutyl ethane) which vaporizes during the spray-drying process, leaving behind generally hollow, porous aerodynamically light microspheres. Other suitable liquid blowing agents include nonfluorinated oils, chloroform, Freons, ethyl acetate, alcohols, hydrocarbons, nitrogen, and carbon dioxide gases.

Although the particulate compositions are preferably formed using a blowing agent as described above, it will be appreciated that, in some instances, no additional blowing agent is required and an aqueous dispersion of the medicament and/or excipients and surfactant(s) are spray dried directly. In such cases, the pharmaceutical formulation may possess special physicochemical properties (e.g., high crystallinity, elevated melting temperature, surface activity, etc.) that makes it particularly suitable for use in such techniques.

In one version, the pharmaceutical formulation is formed by spray drying a feedstock suspension. The first step in the particulate production typically comprises feed stock preparation. If the phospholipid based particulate is intended to act as a carrier for an insoluble active agent, the selected active agent is introduced into a liquid, such as water, to produce a concentrated solution or suspension. The polyvalent cation may be added to the active agent solution or may be added to the phospholipid emulsion as discussed below. The active agent may also be dispersed directly in the emulsion. Alternatively, the active agent may be incorporated in the form of a solid particulate dispersion. The concentration of the active agent used is dependent on the amount of agent required in the final powder and the performance of the delivery device employed. In one version, a polyvalent cation-containing oil-in-water emulsion is then formed in a separate vessel. The oil employed is preferably a fluorocarbon (e.g., distearoyl phosphatidylcholine, perfluorooctyl bromide, perfluorooctyl ethane, perfluorodecalin) which is emulsified with a phospholipid. For example, polyvalent cation and phospholipid may be homogenized in hot distilled water (e.g., 60.degree. C.) using a suitable high shear mechanical mixer (e.g., Ultra-Turrax model T-25 mixer) at 8000 rpm for 2 to 5 minutes. Typically 5 to 25 g of fluorocarbon is added dropwise to the dispersed surfactant solution while mixing. The resulting polyvalent cation-containing perfluorocarbon in water emulsion is then processed using a high pressure homogenizer to reduce the particle size. Typically the emulsion is processed at 12,000 to 18,000 psi, 5 discrete passes and kept at 50 to 80.degree. C. The active agent and perfluorocarbon emulsion are then fed into the spray dryer.

Operating conditions such as inlet and outlet temperature, feed rate, atomization pressure, flow rate of the drying air, and nozzle configuration can be adjusted in order to produce the required particle size, and production yield of the resulting dry particles. Exemplary settings are as follows: an air inlet temperature between 60.degree. C. and 170.degree. C.; an air outlet between 40.degree. C. to 120.degree. C.; a feed rate between 3 ml to about 15 ml per minute; and an aspiration air flow of 300 L/min. and an atomization air flow rate between 25 to 50 L/min. The use of the described method provides for the formation of hollow and/or porous microstructures that are aerodynamically light microparticles with particle diameters appropriate for aerosol deposition into the lung, as discussed above.

Particulate compositions useful in the present invention may alternatively be formed by lyophilization. Lyophilization is a freeze-drying process in which water is sublimed from the composition after it is frozen. The particular advantage associated with the lyophilization process is that biologicals and pharmaceuticals that are relatively unstable in an aqueous solution can be dried without elevated temperatures, and then stored in a dry state where there are few stability problems. With respect to the instant invention such techniques are particularly compatible with the incorporation of peptides, proteins, genetic material and other natural and synthetic macromolecules in particulate compositions without compromising physiological activity. The lyophilized cake containing a fine foam-like structure can be micronized using techniques known in the art to provide the desired sized particles.

In one version, the pharmaceutical formulation is composed of hollow and/or porous microstructures having a bulk density less than 0.5 g/cm$^3$, more preferably less than 0.3 g/cm$^3$, more preferably less than 0.2 g/cm$^3$, and sometimes less 0.1 g/cm$^3$. By providing particles with very low bulk density, the minimum powder mass that can be filled into a unit dose container is reduced, which eliminates the need for carrier particles. That is, the relatively low density of the powders of the present invention provides for the reproducible administration of relatively low dose pharmaceutical compounds. Moreover, the elimination of carrier particles will potentially minimize throat deposition and any "gag" effect, since the large lactose particles will impact the throat and upper airways due to their size.

The powder pharmaceutical formulation may be administered using an aerosolization device. The aerosolization device may be a nebulizer, a metered does inhaler, a liquid dose instillation device, or a dry powder inhaler. The powder pharmaceutical formulation may be delivered by a nebulizer as described in WO 99/16420, by a metered dose inhaler as described in WO 99/16422, by a liquid dose instillation apparatus as described in WO 99/16421, and by a dry powder inhaler as described in U.S. Patent Application Serial Number 09/888,311 filed on June 22, 2001, in WO 02/83220, in U.S. Patent 6,546,929, and in U.S. Patent Application Serial No. 10/616,448 filed on July 8, 2003, all of these patents and patent applications being incorporated herein by reference in their entireties.

In one version, the pharmaceutical formulation is in dry powder form and is contained within a unit dose receptacle which may be inserted into or near the aerosolization apparatus to aerosolize the unit dose of the pharmaceutical formulation. This version is useful in that the dry powder form may be stably stored in its unit dose receptacle for a long period of time. In addition, this version is convenient in that no refrigeration or external power source is required for aerosolization.

In some instances, it is desirable to deliver a unit dose, such as doses of 5 mg or greater of active agent to the lung in a single inhalation. The above described phospholipid hollow and/or porous dry powder particulates allow for doses of 5 mg or greater, often greater than 10 mg, and sometimes greater than 25 mg, to be delivered in a single inhalation and in an advantageous manner. To achieve this, the bulk density of the powder is preferably less than 0.5 g/cm$^3$, and more preferably less than 0.2 g/cm$^3$. Generally, a drug loading of more than 5%, more preferably more than 10%, more preferably more than 20%, more preferably more than 30%, and most preferably more than 40% is also desirable when the required lung dose in more than 5 mg.

These unit dose pharmaceutical formulations may be contained in a capsule that may be inserted into an aerosolization device. The capsule may be of a suitable shape, size, and material to contain the pharmaceutical formulation and to provide the pharmaceutical formulation in a usable condition. For example, the capsule may comprise a wall which comprises a material that does not adversely react with the pharmaceutical formulation. In addition, the wall may comprise a material that allows the capsule to be opened to allow the pharmaceutical formulation to be aerosolized. In one version, the wall comprises one or more of gelatin, hydroxypropyl methylcellulose (HPMC), polyethyleneglycol-compounded HPMC, hydroxyproplycellulose, agar, or the like. In one version, the capsule may comprise telescopically adjoining sections, as described for example in U.S. Patent 4,247,066 which is incorporated herein by reference in its entirety. The size of the capsule may be selected to adequately contain the dose of the pharmaceutical formulation. The sizes generally range from size 5 to size 000 with the outer diameters ranging from about 4.91 mm to 9.97 mm, the heights ranging from about 11.10 mm to about 26.14 mm, and the volumes ranging from about 0.13 ml to about 1.37 ml, respectively. Suitable capsules are available commercially from, for example, Shionogi Qualicaps Co. in Nara, Japan and Capsugel in Greenwood, South Carolina. After filling, a top portion may be placed over the bottom portion to form the a capsule shape and to contain the powder within the capsule, as described in U.S. Patent 4,846,876, U.S. Patent 6,357,490, and in the PCT application WO 00/07572 published on February 17, 2000, all of which are incorporated herein by reference in their entireties.

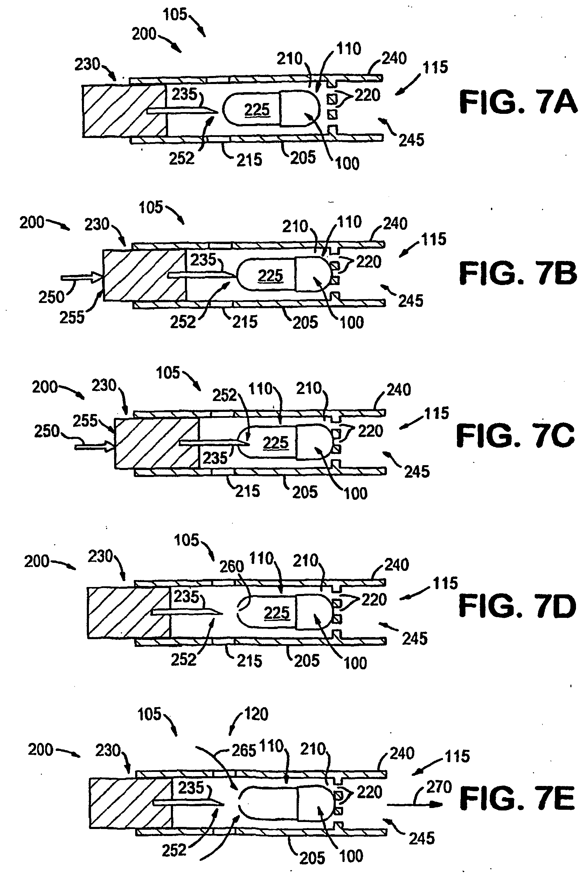

An example of a dry powder aerosolization apparatus particularly useful in aerosolizing a pharmaceutical formulation 100 according to the present invention is shown schematically in Figure 7A. The aerosolization apparatus 200 comprises a housing 205 defining a chamber 210 having one or more air inlets 215 and one or more air outlets 220. The chamber 210 is sized to receive a capsule 225 which contains an aerosolizable pharmaceutical formulation. A puncturing mechanism 230 comprises a puncture member 235 that is moveable within the chamber 210. Near or adjacent the outlet 220 is an end section 240 that may be sized and shaped to be received in a user's mouth or nose so that the user may inhale through an opening 245 in the end section 240 that is in communication with the outlet 220.

The dry powder aerosolization apparatus 200 utilizes air flowing through the chamber 210 to aerosolize the pharmaceutical formulation in the capsule 225. For example, Figures 7A through 7E illustrate the operation of a version of an aerosolization apparatus 200 where air flowing through the inlet 215 is used to aerosolize the pharmaceutical formulation and the aerosolized pharmaceutical formulation flows through the outlet 220 so that it may be delivered to the user through the opening 245 in the end section 240. The dry powder aerosolization apparatus 200 is shown in its initial condition in Figure 7A. The capsule 225 is positioned within the chamber 210 and the pharmaceutical formulation is contained within the capsule 225.

To use the aerosolization apparatus 200, the pharmaceutical formulation in the capsule 225 is exposed to allow it to be aerosolized. In the version of Figures 7A though 7E, the puncture mechanism 230 is advanced within the chamber 210 by applying a force 250 to the puncture mechanism 230. For example, a user may press against a surface 255 of the puncturing mechanism 230 to cause the puncturing mechanism 230 to slide within the housing 205 so that the puncture member 235 contacts the capsule 225 in the chamber 210, as shown in Figure 7B. By continuing to apply the force 250, the puncture member 235 is advanced into and through the wall of the capsule 225, as shown in Figure 7C. The puncture member may comprise one or more sharpened tips 252 to facilitate the advancement through the wall of the capsule 225. The puncturing mechanism 230 is then retracted to the position shown in Figure 7D, leaving an opening 260 through the wall of the capsule 225 to expose the pharmaceutical formulation in the capsule 225.

Air or other gas then flows through an inlet 215, as shown by arrows 265 in Figure 7E. The flow of air causes the pharmaceutical formulation to be aerosolized. When the user inhales 270 through the end section 240 the aerosolized pharmaceutical formulation is delivered to the user's respiratory tract. In one version, the air flow 265 may be caused by the user's inhalation 270. In another version, compressed air or other gas may be ejected into the inlet 215 to cause the aerosolizing air flow 265.

A specific version of a dry powder aerosolization apparatus 200 is described in U.S. Patent 4,069,819 and in U.S. Patent 4,995,385, both of which are incorporated herein by reference in their entireties. In such an arrangement, the chamber 210 comprises a longitudinal axis that lies generally in the inhalation direction, and the capsule 225 is insertable lengthwise into the chamber 210 so that the capsule's longitudinal axis may be parallel to the longitudinal axis of the chamber 210. The chamber 210 is sized to receive a capsule 225 containing a pharmaceutical formulation in a manner which allows the capsule to move within the chamber 210. The inlets 215 comprise a plurality of tangentially oriented slots. When a user inhales through the endpiece, outside air is caused to flow through the tangential slots. This airflow creates a swirling airflow within the chamber 210. The swirling airflow causes the capsule 225 to contact a partition and then to move within the chamber 210 in a manner that causes the pharmaceutical formulation to exit the capsule 225 and become entrained within the swirling airflow. This version is particularly effective in consistently aerosolizing high doses if the pharmaceutical formulation. In one version, the capsule 225 rotates within the chamber 210 in a manner where the longitudinal axis of the capsule is remains at an angle less than 80 degrees, and preferably less than 45 degrees from the longitudinal axis of the chamber. The movement of the capsule 225 in the chamber 210 may be caused by the width of the chamber 210 being less than the length of the capsule 225. In one specific version, the chamber 210 comprises a tapered section that terminates at an edge. During the flow of swirling air in the chamber 210, the forward end of the capsule 225 contacts and rests on the partition and a sidewall of the capsule 225 contacts the edge and slides and/or rotates along the edge. This motion of the capsule is particularly effective in forcing a large amount of the pharmaceutical formulation through one or more openings 260 in the rear of the capsule 225.

In another version, the dry powder aerosolization apparatus 200 may be configured differently than as shown in Figures 7A through 7E. For example, the chamber 210 may be sized and shaped to receive the capsule 225 so that the capsule 225 is orthogonal to the inhalation direction, as described in U.S. Patent 3,991,761. As also described in U.S. Patent 3,991,761, the puncturing mechanism 230 may puncture both ends of the capsule 225. In another version, the chamber may receive the capsule 225 in a manner where air flows through the capsule 225 as described for example in U.S. Patent 4,338,931 and in U.S. Patent 5,619,985. In another version, the aerosolization of the pharmaceutical formulation may be accomplished by pressurized gas flowing through the inlets, as described for example in US Patent 5,458,135, U.S. Patent 5,785,049, and U.S. Patent 6,257,233, or propellant, as described in PCT Publication WO 00/72904 and U.S. Patent 4,114,615. All of the above references being incorporated herein by reference in their entireties.

The pharmaceutical formulation disclosed herein may also be administered to the nasal or pulmonary air passages of a patient via aerosolization, such as with a metered dose inhaler. The use of such stabilized preparations provides for superior dose reproducibility and improved lung deposition as disclosed in WO 99/16422, hereby incorporated in its entirety by reference. MDIs are well known in the art and could easily be employed for administration of the claimed dispersions without undue experimentation. Breath activated MDIs, as well as those comprising other types of improvements which have been, or will be, developed are also compatible with the stabilized dispersions and present invention and, as such, are contemplated as being within the scope thereof. However, it should be emphasized that, in preferred embodiments, the stabilized dispersions may be administered with an MDI using a number of different routes including, but not limited to, topical, nasal, pulmonary or oral. Those skilled in the art will appreciate that, such routes are well known and that the dosing and administration procedures may be easily derived for the stabilized dispersions of the present invention.

Along with the aforementioned embodiments, the stabilized dispersions of the present invention may also be used in conjunction with nebulizers as disclosed in PCT WO 99/16420, the disclosure of which is hereby incorporated in its entirety by reference, in order to provide an aerosolized medicament that may be administered to the pulmonary air passages of a patient in need thereof. Nebulizers are well known in the art and could easily be employed for administration of the claimed dispersions without undue experimentation. Breath activated nebulizers, as well as those comprising other types of improvements which have been, or will be, developed are also compatible with the stabilized dispersions and present invention and are contemplated as being with in the scope thereof.

Along with DPIs, MDIs and nebulizers, it will be appreciated that the stabilized dispersions of the present invention may be used in conjunction with liquid dose instillation or LDI techniques as disclosed in, for example, WO 99/16421 hereby incorporated in its entirety by reference. Liquid dose instillation involves the direct administration of a stabilized dispersion to the lung. In this regard, direct pulmonary administration of bioactive compounds is particularly effective in the treatment of disorders especially where poor vascular circulation of diseased portions of a lung reduces the effectiveness of intravenous drug delivery. With respect to LDI the stabilized dispersions are preferably used in conjunction with partial liquid ventilation or total liquid ventilation. Moreover, the present invention may further comprise introducing a therapeutically beneficial amount of a physiologically acceptable gas (such as nitric oxide or oxygen) into the pharmaceutical microdispersion prior to, during or following administration.

It will be appreciated that the particulate compositions disclosed herein comprise a structural matrix that exhibits, defines or comprises voids, pores, defects, hollows, spaces, interstitial spaces, apertures, perforations or holes. The absolute shape (as opposed to the morphology) of the perforated microstructure is generally not critical and any overall configuration that provides the desired characteristics is contemplated as being within the scope of the invention. Accordingly, preferred embodiments can comprise approximately microspherical shapes. However, collapsed, deformed or fractured particulates are also compatible.

In accordance with the teachings herein the particulate compositions will preferably be provided in a "dry" state. That is the particulates will possess a moisture content that allows the powder to remain chemically and physically stable during storage at ambient temperature and easily dispersible. As such, the moisture content of the microparticles is typically less than 6% by weight, and preferably less 3% by weight. In some instances the moisture content will be as low as 1% by weight. The moisture content is, at least in part, dictated by the formulation and is controlled by the process conditions employed, e.g., inlet temperature, feed concentration, pump rate, and blowing agent type, concentration and post drying. Reduction in bound water leads to significant improvements in the dispersibility and flowability of phospholipid based powders, leading to the potential for highly efficient delivery of powdered lung surfactants or particulate composition comprising active agent dispersed in the phospholipid. The improved dispersibility allows simple passive DPI devices to be used to effectively deliver these powders.

Although the powder compositions are preferably used for inhalation therapies, the powders of the present invention can also be administered by other techniques known in the art, including, but not limited to oral, intramuscular, intravenous, intratracheal, intraperitoneal, subcutaneous, and transdermal, either as capsules, tablets, dry powders, reconstituted powders, or suspensions.

Accordingly, one embodiment of the present invention is directed to oral administration of the particulate compositions. According to this embodiment, the composition of the present invention are provided in unit dose form, such as capsules, caplets, tablets, lozenges and the like or are suspended in a suitable vehicle for subsequent administration as a liquid suspension. The fine powders of the present invention have a large surface area, thus providing immediate release formulations.

According to another embodiment, release kinetics of the active agent containing composition is controlled. According to a preferred embodiment, the compositions of the present invention provide immediate release due to the small size of the insoluble active agent, i.e. less than 3 microns, incorporated into the matrix material. Alternatively, the compositions of the present invention may be provided as non-homogeneous mixtures of active agent incorporated into a matrix material and unincorporated active agent in order to provide desirable release rates of active agent. According to this embodiment, active agents formulated using the emulsion-based manufacturing process of the present invention have utility in immediate release applications when administered via either pulmonary or oral routes. Rapid release is facilitated by: (a) the high surface area of the low density porous powders; (b) the small size of the drug crystals that are incorporated therein, and; (c) the low surface energy of the particles resulting from the lack of long-range order for the phospholipids on the surface of the particles.

The nature of the surface packing of phospholipid molecules is influenced by the nature of their packing in the spray-drying feedstock and the drying conditions and other formulation components utilized. In the case of spray-drying of active agents solubilized within a small unilamellar vesicle (SUV) or multilamellar vesicle (MLV), the active remains encapsulated within multiple bilayers with a high degree of long-range order over fairly large length scales. In this case, the spray-dried formulation may exhibit sustained release characteristics.

In contrast, spray-drying of a feedstock comprised of emulsion droplets and dispersed or dissolved active in accordance with the teachings herein leads to a phospholipid matrix with less long-range order, thereby facilitating rapid release. While not being bound to any particular theory, it is believed that this is due in part to the fact that the active is never formally encapsulated in the phospholipid, and the fact that the phospholipid is initially present on the surface of the emulsion droplets as a monolayer (not a bilayer as in the case of liposomes). The higher degree of disorder observed in spray-dried particles prepared by the emulsion-based manufacturing process of the present invention is reflected in very low surface energies, where values as low as 20 mN/m have been observed for spray-dried DSPC particles (determined by inverse gas chromatography). Small angle X-ray scattering (SAXS) studies conducted with spray-dried phospholipid particles have also shown a high degree of disorder for the lipid, with scattering peaks smeared out, and length scales extending in some instances only beyond a few nearest neighbors.

It should be noted that having a high gel to liquid crystal phase transition temperature is not sufficient in itself in achieving sustained release. Having a sufficient length scale for the bilayer structures is also important. To facilitate rapid release, an emulsion-system of high porosity (high surface area), and no interaction between the drug substance and phospholipid is preferred. The pharmaceutical formulation formation process may also include the additions of other formulation components (e.g., small polymers such as Pluronic F-68; carbohydrates, salts, hydrotropes) to break the bilayer structure are also contemplated.

To achieve a sustained release, incorporation of the phospholipid in bilayer form is preferred, especially if the active agent is encapsulated therein. In this case increasing the Tm of the phospholipid may provide benefit via incorporation of divalent counterions or cholesterol. As well, increasing the interaction between the phospholipid and drug substance via the formation of ion-pairs (negatively charged active + steaylamine, postitively charged active + phosphatidylglycerol) would tend to decrease the dissolution rate. If the active is amphiphilic, surfactant/surfactant interactions may also slow active dissolution.

The addition of divalent counterions (e.g. calcium or magnesium ions) to long-chain saturated phosphatidylcholines results in an interaction between the negatively charged phosphate portion of the zwitterionic headgroup and the positively charged metal ion. This results in a displacement of water of hydration and a condensation of the packing of the phospholipid lipid headgroup and acyl chains. Further, this results in an increase in the Tm of the phospholipid. The decreases in headgroup hydration can have profound effects on the spreading properties of spray-dried phospholipid particles on contact with water. A fully hydrated phosphatidylcholine molecule will diffuse very slowly to a dispersed crystal via molecular diffusion through the water phase. The process is exceedingly slow because the solubility of the phospholipid in water is very low (ca., $10^{-10}$ mol/L for DPPC). Prior art attempts to overcome this phenomena include homogenizing the crystals in the presence of the phospholipid. In this case, the high degree of shear and radius of curvature of the homogenized crystals facilitates coating of the phospholipid on the crystals. In contrast, "dry" phospholipid powders according to this invention can spread rapidly when contacted with an aqueous phase, thereby coating dispersed crystals without the need to apply high energies. For example, the surface tension of spray-dried DSPC/Ca mixtures at the air/water interface decreases to equilibrium values (ca., 20mN/m) as fast as a measurement can be taken. In contrast, liposomes of DSPC decrease the surface tension (ca., 50 mN/m) very little over a period of hours, and it is likely that this reduction is due to the presence of hydrolysis degradation products such as free fatty acids in the phospholipid. Single-tailed fatty acids can diffuse much more rapidly to the air/water interface than can the hydrophobic parent compound. Hence the addition of calcium ions to phosphatidylcholines can facilitate the rapid encapsulation of crystalline drugs more rapidly and with the lower applied energy.

In another version, the pharmaceutical formulation comprises low density particulates achieved by co-spray-drying nanocrystals with a perfluorocarbon-in-water emulsion.

The foregoing description will be more fully understood with reference to the following Examples. Such Examples, are, however, merely representative of preferred methods of practicing the present invention and should not be read as limiting the scope of the invention.

Example I

Preparation of Spray-Dried Amphotericin B Particles

Amphotericin particles were prepared by a two-step process. In the first step, 10.52 g of amphotericin B (Alpharma, Copenhagen, Denmark), 10.12 g of distearoyl phosphatidylcholine (DSPC) (Genzyme, Cambridge, MA), and 0.84 g calcium chloride (JT Baker, Phillipsburg, NJ) were dispersed in 1045 g of hot deionized water (T = 70°C) using an Ultra-Turrax mixer (model T-25) at 10,000 rpm for 2 to 5 minutes. Mixing was continued until the DSPC and amphotericin B appeared visually to be dispersed.

381 g of perfluorooctyl ethane (PFOE) was then added slowly at a rate of approximately 50-60 ml/min during mixing After the addition was complete, the emulsion/drug dispersion was mixed for an additional period of not less than 5 minutes at 12,000 rpm. The coarse emulsion was then passed through a high pressure homogenizer (Avestin, Ottawa, Canada) at 12,000 - 18,000 psi for 3 passes, followed by 2 passes at 20,000 - 23,000 psi.

The resulting fine emulsion was utilized as the feedstock in for the second step, i.e. spray-drying on a Niro Mobile Minor. The following spray conditions were employed: total flow rate = 70 SCFM, inlet temperature = 110°C, outlet temperature = 57°C, feed pump=38 mL min$^{-1}$, atomizer pressure = 105 psig, atomizer flow rate = 12 SCFM.

A free flowing pale yellow powder was collected using a cyclone separator. The collection efficiency of the amphotericin B formulation was 60%. The geometric diameter of the amphotericin B particles was confirmed by laser diffraction (Sympatech Helos H1006, Clausthal-Zellerfeld, Germany), where a volume weighted mean diameter (VMD) of 2.44 $\mu$m was found. Scanning electron microscopy (SEM) analysis showed the powders to be small porous particles with high surface roughness. There was no evidence of any unincorporated AmB crystals in the 5 SEM views provided for each collector. Differential scanning calorimetry analysis of the dry particles revealed the $t_m$ for the amphotericin B in the powder to be 78°C, which is similar to what is observed for spray-dried neat material.

Example II

Aerosol Performance for Spray-Dried Amphotericin B Particles

The resulting dry amphotericin B particles prepared in Example I were hand filled into #2 HPMC capsules (Shionogi, Japan) and allowed to equilibrate at 15% - 20% RH overnight. A fill mass of approximately 10 mg was used, which represented approximately ½ the fill volume of the #2 capsule.

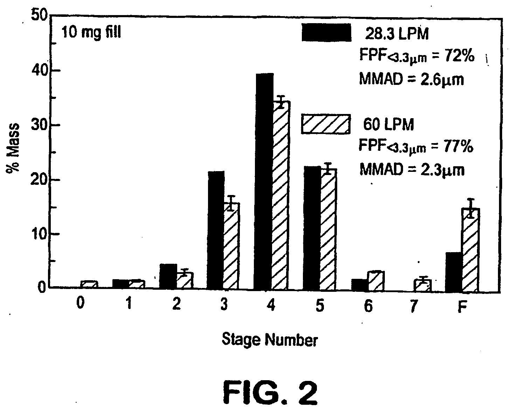

Aerodynamic particle size distributions were determined gravimetrically on an Andersen cascade impactor (ACI). Particle size distributions were measured at flow rates of 28.3 L·min$^{-1}$ (i.e., comfortable inhalation effort) and 56.6 L·min$^{-1}$ (i.e., forceful inhalation effort) using the Turbospin DPI device. A total volume of 2 liters was drawn through the device. At the higher flow rate, two ACIs were used in parallel at a calibrated flow rate of 28.3 L·min$^{-1}$ and a total flow through the device of 56.6 L·min$^{-1}$. In both cases the set-up represents conditions at which the ACI impactor plates are calibrated. Excellent aerosol characteristics was observed as evidenced by a MMAD less than 2.6 $\mu$m and FPF$_{<3.3\mu m}$ greater than 72%. The effect of flow rate on performance was also assessed (Figure 2) using the Turbospin® (PH&T, Italy) DPI device operated at 56.6 L min$^{-1}$ into 2 ACIs used in parallel. No significant difference in the deposition profile was observed at the higher flow rates, demonstrating minimal flow rate dependant performance. This abovementioned example illustrates the aerosol performance of the present powder is independent of flow rate which should lead to more reproducible patient dosing.

Example III

Effect of Stability Storage on Aerosol Performance of Spray-Dried Amphotericin B Particles.

The resulting dry amphotericin B particles prepared in Example I were hand filled into #2 HPMC capsules (Shionogi, Japan) and allowed to equilibrate at 15% - 20% RH overnight. A fill mass of approximately 10 mg was used, which represented approximately ½ the fill volume of the #2 capsule. The filled capsules were placed in individually indexed glass vials that were packaged in laminated foil-sealed pouch and subsequently stored at 25°C/60%RH or 40°C/75%RH.

Emitted dose (ED) measurements were performed using the Turbospin® (PH&T, Italy) DPI device, described in U.S. Patent 4,069,819 and in U.S. Patent 4,995,385, operated at its optimal sampling flow rate of 60 L·min$^{-1}$, and using a total volume of 2 liters. A total of 10 measurements was determined for each storage variant.

The aerodynamic particle size distributions were determined gravimetrically on an Andersen cascade impactor (ACI). Particle size distributions were measured at flow rates of 28.3 L·min$^{-1}$ using the Turbospin® DPI device and using a total volume of 2 liters.

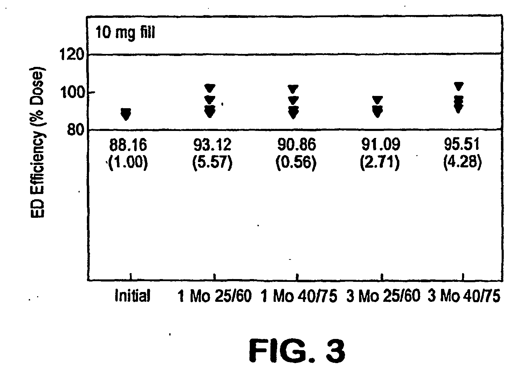

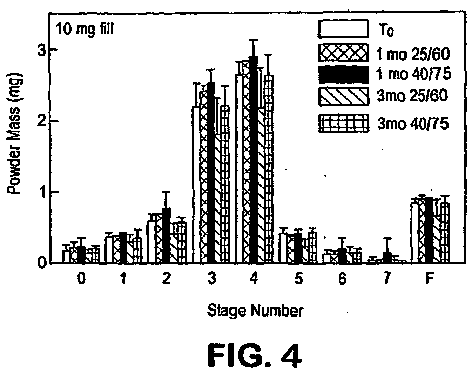

Excellent aerosol characteristics was observed as evidenced by a mean ED of 93% +/- 5.3%, MMAD = 2.6$\mu$m and FPF$_{<3.3\mu m}$ = 72% (Figures 3 and 4). No significant change in aerosol performance (ED, MMAD or FPF) was observed after storage at elevated temperature and humidity, demonstrating excellent stability characteristics. The current specifications ED performance stipulates that >90% of the delivered doses be within ±25% of the label claim, with less than 10% of the doses ±35%. A recent draft guidance published by the FDA [10] proposes that the limits be tightened, such that >90% of the delivered doses be within ±20% of the label claim, with none outside of ±25%. Statistically speaking, an RSD of 6% would be required to meet the proposed FDA specifications.

Not only are the results of the foregoing example within the current guidelines, but they are also within the limits of the proposed guidelines, a strong testament to the excellent dispersibility, aerosol characteristics and stability afforded by this formulation.

Example IV

Spray-Dried Amphotericin B Particles Comprised of Various Phosphatidylcholines

Spray-dried particles comprising approximately 50% amphotericin B were prepared using various phosphatidylcholines (PC) as the surfactant following the two-step process described in Example I. Formulations were prepared using DPPC (Genzyme, Cambridge, MA), DSPC (Genzyme, Cambridge, MA) and SPC-3 (Lipoid KG, Ludwigshafen, Germany) The feed solution was prepared using the identical equipment and process conditions described therein. The 50% amphotericn B formulation is as follows:

| | |
|---|---|
| Amphotericin B | 0.733g |
| PC | 0.714g |
| CaCl$_2$ | 60 mg |
| PFOB | 32 g |
| DI water | 75 g |

The resulting multi-particulate emulsion was utilized as the feedstock in for the second step, i.e. spray-drying on a B-191 Mini Spray-Drier (Büchi, Flawil, Switzerland). The following spray conditions were employed: aspiration=100%, inlet temperature=85°C, outlet temperature=60°C, feed pump=1.9 mL min$^{-1}$, atomizer pressure=60-65 psig, atomizer flow rate=30-35 cm. The aspiration flow (69-75%) was adjusted to maintain an exhaust bag pressure of 30-31 mbar. Free flowing yellow powders were collected using a standard cyclone separator. The geometric diameter of the amphotericin B particles was confirmed by laser diffraction (Sympatech Helos H1006, Clausthal-Zellerfeld, Germany), where a volume weighted mean diameters (VMD) were found to be similar and ranged from 2.65 $\mu$m to 2.75 $\mu$m. Scanning electron microscopy (SEM) analysis showed the powders to be small porous particles with high surface roughness.

Aerodynamic particle size distributions were determined gravimetrically on an Andersen cascade impactor (ACI). Particle size distributions were measured at flow rates of 56.6 L·min$^{-1}$ (i.e., forceful inhalation effort) using the Turbospin DPI device. A total volume of 2 liters was drawn through the device. Two ACIs were used in parallel at a calibrated flow rate of 28.3 L·min$^{-1}$ and a total flow through the devices of 56.6 L·min$^{-1}$. Similar aerosol characteristics were observed in the amphotericin B produced with the 3 types of phosphatidylcholines, with MMADs less than 2.5$\mu$m and FPF$_{<3.3\mu m}$ greater than 72%. This abovementioned example illustrates the flexibility of the formulation technology to produce amphotericin B powders independent of the type of phosphatidylcholie employed.

Example V

Preparation of 70% Amphotericin B Spray-Dried Particles

Amphotericin particles were prepared following the two-step process described in Example I. The feed solution was prepared using the identical equipment and process conditions described therein. The 70% amphotericin B formulation is as follows:

| | |
|---|---|
| Amphotericin B | 0.70g |
| DSPC | 0.265g |
| CaCl$_2$ | 24 mg |
| PFOB | 12 g |
| DI water | 35 g |

The resulting multi-particulate emulsion was utilized as the feedstock in for the second step, i.e. spray-drying on a B-191 Mini Spray-Drier (Büchi, Flawil, Switzerland). The following spray conditions were employed: aspiration=100%, inlet temperature=85°C, outlet temperature=60°C, feed pump=1.9 mL min$^{-1}$, atomizer pressure=60-65 psig, atomizer flow rate=30-35 cm. The aspiration flow (69-75%) was adjusted to maintain an exhaust bag pressure of 30-31 mbar. A free flowing yellow powder was collected using a standard cyclone separator. The geometric diameter of the amphotericin B particles was confirmed by laser diffraction (Sympatech Helos H1006, Clausthal-Zellerfeld, Germany), where a volume weighted mean diameter (VMD) of 2.96 $\mu$m was found. Scanning electron microscopy (SEM) analysis showed the powders to be small porous particles with high surface roughness. This foregoing example illustrates the flexibility of the present powder engineering technology to produce high amphotericin B content using the herein described multi-particulate approach.

Example VI

Aerosol Performance of Spray-Dried Amphotericin B Particles in Various DPI Devices.

The resulting dry amphotericin B particles prepared in Example V were hand filled into #2 HPMC (Shionogi, Japan) or #3 (Capsugel, Greenwood, SC) capsules and allowed to equilibrate at 15% - 20% RH overnight. A fill mass of approximately 10 mg was used, which represents approximately ½ the fill volume for a #2 capsule or 5/8 for a #3 capsule. The aerosol characteristics were examined using a Turbospin® (PH&T, Italy), Eclipse® (Aventis, UK) and Cyclohaler® (Novartis, Switzerland) DPI devices. The Cyclohaler utilizes a # 3 capsule, whereas the Turbospin and Cyclohaler utilize size # 2 capsules

Aerodynamic particle size distributions were determined gravimetrically on an Andersen cascade impactor (ACI). Particle size distributions were measured at a flow rate 56.6 L·min$^{-1}$ which represents a forceful inhalation effort for both Turbospin and Eclipse DPI devices and comfortable for Cyclohaler. A total volume of 2 liters was drawn through the device. Two ACIs were used in parallel at a calibrated flow rate of 28.3 L·min$^{-1}$ and a total flow through the devices of 56.6 L·min$^{-1}$. Similar aerosol characteristics were observed in all devices as evidenced by a MMAD less than 2.5 $\mu$m and FPF$_{<3.3\mu m}$ greater than 71%. This abovementioned example illustrates the aerosol performance of the present powder is independent of device design with medium and low resistance and capsule size speaks volumes to the dispersibility of the amphotericin B powder tested.

Example VII

Formulation of an inhaled Molecule B

Unnamed Molecule B, having a water solubility of 0.14 mg/ml (20 °C) was formulated for an aerosolizable pharmaceutical formulation. The homogenized crystals had a median diameter of 1.5 microns (laser diffraction), and the characteristic time for Ostwald ripening was estimated to be about 3 hours.

A perfluorocarbon emulsion was first prepared by adding a 2:1 molar ratio of distearoylphosphatidylcholine (DSPC) : calcium chloride to hot DI water (>70°C) and adding the blowing agent, perfluorooctyl bromide (PFOB), if required, while dispersing with an Ultra Turrax mixer. This emulsion was then homogenized under high pressure using an EmulsiFlex-C5 Homogenizer. The material was passed through the homogenizer two times at 12-16 Kpsi. The emulsion was then chilled in an ice-water bath to about 5 °C. Molecule B, as received, was then added to the emulsion. This suspension was stirred vigorously on a stirring plate in an ice-water bath for approximately three minutes and then homogenized using an EmulsiFlex-C5 Homogenizer. The material was passed through the homogenizer three times jacketed with a circulating ice-water bath, one time at 14-18 Kpsi and two times at 18-22 Kpsi. The suspension was stirred continuously in an ice-water bath before and during spray drying.

During the spray drying of the suspension formulations, processing conditions (temperature, pressure, solids content, and blowing agent concentration) were altered to modulate the size of the primary particles formed.

An experiment was conducted to assess the stability of the spray-drying feedstock (emulsion + microcrystals) to variations in storage time and temperature. The stability was assessed by measurements of the median particle size of the spray-dried particulates by laser diffraction (Sympatec) (Table 1), their aerodynamic size via Andersen cascade impaction (Table 2), and the drug content and purity. No differences in physical or chemical stability were noted after storage of the feedstock for 72 hr at ambient conditions. The characteristic ripening time was estimated to be approximately 3 hr. Hence, the long-term stability of the feedstock is unexpected with respect to predictions of Ostwald ripening processes in these complex systems.

Table 1

| Lot | Hold Duration / Temperature | X50 ($\mu$m) (powder) | Purity (%) | Drug Content (mg/mg) |
|-----|-----------------------------|-----------------------|------------|----------------------|
| 1 | 0 hr | 2.16 | 97.18 | 0.67 |
| 2 | 24 hr at 2-8 °C | 2.12 | 97.30 | 0.68 |
| 3 | 72 hr at 2-8 °C | 2.28 | 97.21 | 0.66 |
| 4 | 72 hr at ambient | 2.34 | 97.20 | 0.68 |

Table 2

| | | | | |
|---|---|---|---|---|
| **Gravimetric Aerosol Particle Size** | | | | |
| Lot | Storage duration /temperature | MMAD ($\mu$m) | Efficiency (%) | FPD$_{3.3\ \mu m}$ (mg) |
| 1 | 0 hr | 3.1 | 77 | 7.0 |
| 2 | 24 hr at 2-8 °C | 3.1 | 77 | 6.9 |
| 3 | 72 hr at 2-8 °C | 3.3 | 77 | 6.3 |
| 4 | 72 hr at ambient | 3.3 | 73 | 6.1 |
| **Drug Specific Aerosol Particle Size** | | | | |
| Lot | Storage duration /temperature | MMAD ($\mu$m) | Efficiency (%) | FPD$_{3.3\ \mu m}$ (mg) |
| 1 | 0 hr | 3.0 | 76 | 7.0 |
| 2 | 24 hr at 2-8 °C | 3.1 | 76 | 6.9 |
| 3 | 72 hr at 2-8 °C | 3.3 | 74 | 6.4 |
| 4 | 72 hr at ambient | 3.2 | 75 | 6.4 |

It is worth noting that the aerodynamic particle size distributions were equivalent when the particle size distributions were quantitated either gravimetrically or with a drug specific method. This implies that the ca., 1.5 $\mu$m median sized crystals (as obtained by laser diffraction, Malvern) are evenly distributed across the distribution of aerodynamic particle sizes.

The physical stability of the spray-dried powders was also assessed. No significant differences in emitted dose (from the passive Turbospin inhaler at 60 L/min), MMAD or FPD were noted following storage at 25 °C/60% RH or 40 °C/75% RH over a period of 3 months.

Table 3

| Storage condition | Test parameter | Initial | 2 weeks | 1 month | 3 months |
|-------------------|----------------|---------|---------|---------|----------|
| 25 °C/60% RH | %ED (SD) | 86 (1.9) | 85 (1.1) | 84 (3.5) | 85 (2.8) |
| | MMAD ($\mu$m) | 2.4 | 2.5 | 2.5 | 2.5 |

(continued)

| Storage condition | Test parameter | Initial | 2 weeks | 1 month | 3 months |
|---|---|---|---|---|---|
| | $FPD_{3.3\mu m}$ (mg) | 5.7 | 5.9 | 5.5 | 5.6 |
| 40 °C/75% RH | %ED (SD) | 86 (1.9) | 85 (3.2) | 85 (3.5) | 89 (2.0) |
| | MMAD ($\mu$m) | 2.4 | 2.5 | 2.6 | 2.6 |
| | $FPD_{3.3\mu m}$ (mg) | 5.7 | 5.7 | 5.6 | 5.5 |

As well, no loss in chemical stability was noted (Table 4) under the same storage conditions. This is in contrast to lyophilized powders or spray-dried powders where the drug is present in an amorphous state. In these powders significant hydrolysis of the active agent is noted on storage. Maintaining the drug substance in the crystalline state overcomes the chemical stability issues.

Table 4

| Powder | Storage Condition | Total Impurities | | | |
|---|---|---|---|---|---|
| | | t = 0 | T = 0.5 | t = 1 | t = 3 mo |
| Lyophilized target | 40 °C/75% RH, packaged | 3.19 | 3.91 | 4.60 | 5.71 |
| Amorphous API PulmoSphere | 40 °C/75% RH, packaged | 3.90 | 17.8 | n.d. | n.d. |
| Crystalline API PulmoSphere | 40 °C/75% RH, packaged capsules | 2.95 | 2.85 | 2.97 | 2.83 |
| | 40 °C/75% RH, bulk powder | 2.95 | 2.79 | 2.85 | 2.96 |
| | 40 °C/75% RH, capsules | 2.95 | 2.81 | 2.87 | 3.05 |

Example VIII.

Formulation of inhaled Molecule C

Unnamed Molecule C is characterized by a Tg < 20 °C and water solubility C = 0.6 mg/ml (25 °C). Spray-dried lipid formulations of Molecule C were prepared by the emulsion-based manufacturing method described above for Molecule B. Aerodynamic particle size distributions for the spray-dried powders delivered from the Monohaler at a flow rate of 90 L/min were assessed with a multistage liquid impinger. Capsule fill masses ranging from 9 to 28 mg were assessed. Little differences were noted with capsule fill mass, indicating that a $FPD_{5.0\mu m} > 10$ mg could be achieved in a single inhalation.

Table 5

| 90 L/min, monohaler | | | | |
|---|---|---|---|---|
| Capsule Fill Mass (mg) | API Fill Mass (mg) | MMAD ($\mu$m) | $FPF_{5.0\mu m}$ (%) | $FPD_{5.0\mu m}$ (mg) |
| 9.2 | 5.3 | 2.3 | 88 | 4.0 |
| 14.8 | 8.5 | 2.4 | 85 | 6.4 |
| 28.3 | 16.3 | 2.4 | 80 | 11.3 |

No loss in aerosol performance was noted following storage for 2 weeks at 40°C/75%RH as seen in Table 6.

Table 6

| Parameter | Initial | 2 weeks at 40 °C/75%RH |
|---|---|---|
| MMAD ($\mu$m) | 2.3 | 2.5 |
| $FPF_{5.0\mu m}$ (%) | 83 | 76 |
| $FPD_{5.0\mu m}$ (mg) | 10.3 | 10.9 |

Example IX.

Formulation of inhaled Molecule D

The physical characteristics of unnamed Molecule D are: Tg ~10°C, C = 0.53 mg/ml (25 °C). Formulations of Molecule D were prepared by the same method as described above for Molecule B. The formulation was comprised of 61% Molecule D, 36% DSPC, and 3% calcium chloride. Aerosol performance and chemical stability for the drug substance are detailed below. Aerosol performance was measured via Andersen cascade impaction following delivery from the Turbospin device at a flow rate of 60 L/min. The purity of the drug substance and aerosol performance was maintained following storage for 4 weeks at ambient conditions.

Table 7

| Turbospin, 60 L/min | | | | | |
|---|---|---|---|---|---|
| Timepoint | %ED (RSD) | MMAD ($\mu$m) | FPF$_{3.3\mu m}$ (%) | Purity (%) | Cis isomer (%) |
| Initial | 73 (2) | 3.1 | 57 | 99.2 | 0.74 |
| t = 4 weeks, 2.8°C/ambient | 70(4) | 3.1 | 57 | 99.3 | 0.75 |
| t = 4 weeks, 25°C/ambient | 70 (5) | 3.1 | 56 | 99.2 | 0.75 |
| Purity at 2 months (bulk powder refrigerated) = 99.2%, 0.75% cis-isomer | | | | | |

Example X.

Formulation of Molecule E

The aerosol performance of insoluble unnamed Molecule E delivered from the Monohaler device at a flow rate of 90 L/min are captured below. Acceptable aerosol performance and physical stability are noted over 2 weeks under accelerated storage. No changes in chemical purity were noted during processing or on storage.

Table 8

| | Initial | | 25/60 | | 40/75 | |
|---|---|---|---|---|---|---|
| Formulation | MMAD | %<5.0 um | MMAD | %<5.0 um | MMAD | %<5.0 um |
| E1 | 2.8$\mu$m | 81 | 2.5$\mu$m | 91 | 2.6$\mu$m | 90 |
| E2 | 2.3$\mu$m | 89 | 2.3$\mu$m | 93 | 2.4$\mu$m | 94 |
| E3 | 2.6$\mu$m | 87 | 2.6$\mu$m | 89 | 2.8$\mu$m | 88 |
| E4 | 2.5$\mu$m | 91 | 2.4$\mu$m | 91 | 2.3$\mu$m | 96 |
| E5 | 4.5$\mu$m | 54 | 3.2$\mu$m | 72 | 3.1$\mu$m | 79 |
| E6 | 2.5$\mu$m | 97 | 2.7$\mu$m | 90 | 2.6$\mu$m | 92 |
| E7 | 3.1$\mu$m | 66 | n/a | n/a | n/a | n/a |
| E8 | 2.6$\mu$m | 93 | 2.4$\mu$m | 93 | 2.6$\mu$m | 90 |

Although the present invention has been described in considerable detail with regard to certain preferred versions thereof, other versions are possible, and alterations, permutations and equivalents of the version shown will become apparent to those skilled in the art upon a reading of the specification and study of the drawings. For example, the relative positions of the elements in the aerosolization device may be changed, and flexible parts may be replaced by more rigid parts that are hinged, or otherwise movable, to mimic the action of the flexible part. In addition, the passageways need not necessarily be substantially linear, as shown in the drawings, but may be curved or angled, for example. Also, the various features of the versions herein can be combined in various ways to provide additional versions of the present invention. Furthermore, certain terminology has been used for the purposes of descriptive clarity, and not to limit the present invention. Therefore, any appended claims should not be limited to the description of the preferred versions contained herein and should include all such alterations, permutations, and equivalents as fall within the true spirit and scope of the present invention.

**Claims**

1. A pharmaceutical formulation for pulmonary administration, comprising a plurality of particulates having a mass median diameter less than 20 μm, wherein each particulate comprises: (a) a lipid matrix; and (b) a particle of an active agent in the lipid matrix, said active agent in crystalline form and having a solubility in water of less than 1.0 mg/ml, wherein at least 90% of the active agent particles in the formulation have a geometric diameter less than 3 μm.

2. The pharmaceutical formulation of claim 1 wherein the plurality of particulates has a mass median diameter less than 10 μm.

3. The pharmaceutical formulation of claim 2, wherein the plurality of particulates has a mass median diameter less than 5 μm.

4. The pharmaceutical formulation of claim 1, wherein at least at least 95% of the active agent particles have a geometric diameter less than 3 μm.

5. The pharmaceutical formulation of claim 4, wherein at least 50% of the active agent particles have a geometric diameter between 0.5 μm and 3 μm.

6. The pharmaceutical formulation of claim 5, wherein at least 50% of the active agent particles have a geometric diameter between 1 μm and 3 μm.

7. The pharmaceutical formulation of claim 1, wherein the lipid matrix comprises a phospholipid.

8. The pharmaceutical formulation of claim 7, wherein the phospholipid has a gel to liquid phase transition temperature greater than about 40° C.

9. The pharmaceutical formulation of claim 7, wherein the phospholipid is a phosphoglyceride.

10. The pharmaceutical formulation of claim 7, wherein the phospholipid is a saturated phospholipid.

11. The pharmaceutical formulation of claim 7, wherein the particulates further include a polyvalent cation effective to increase the gel-to-liquid transition temperature of the phospholipid.

12. The pharmaceutical formulation of claim 1, wherein the particulates further include a polyvalent cation.

13. The pharmaceutical formulation of claim 12, wherein the polyvalent cation is a divalent cation.

14. The pharmaceutical formulation of claim 13, wherein the divalent cation is $Ca^{2+}$, $Mg^{2+}$, or $Zn^{2+}$.

15. The pharmaceutical formulation of claim 1, wherein the active agent is an antimycotic agent.

16. The pharmaceutical formulation of claim 15, wherein the antimycotic agent comprises one or more of natamycin, amphotericin B, voriconazole, and miconazole.

17. The pharmaceutical formulation of claim 16, wherein the antimycotic agent is amphotericin B.


**Patentansprüche**

1. Pharmazeutische Formulierung zur pulmonalen Verabreichung, umfassend eine Vielzahl von Feststoffteilchen, die einen mittleren Massendurchmesser von weniger als 20 μm haben, wobei jedes Feststoffteilchen umfasst: (a) eine Lipidmatrix; und (b) ein Partikel eines Wirkstoffs in der Lipidmatrix, wobei der Wirkstoff in kristalliner Form vorliegt und eine Löslichkeit in Wasser von weniger als 1,0 mg/ml hat, wobei mindestens 90% der Wirkstoffpartikel in der Formulierung einen geometrischen Durchmesser von weniger als 3 μm haben.

2. Pharmazeutische Formulierung nach Anspruch 1, wobei die Vielzahl der Feststoffteilchen einen mittleren Massendurchmesser von weniger als 10 μm hat.

**3.** Pharmazeutische Formulierung nach Anspruch 2, wobei die Vielzahl der Feststoffteilchen einen mittleren Massendurchmesser von weniger als 5 $\mu$m hat.

**4.** Pharmazeutische Formulierung nach Anspruch 1, wobei mindestens mindestens 95% der Wirkstoffpartikel einen geometrischen Durchmesser von weniger als 3 $\mu$m haben.

**5.** Pharmazeutische Formulierung nach Anspruch 4, wobei mindestens 50% der Wirkstoffpartikel einen geometrischen Durchmesser zwischen 0,5 $\mu$m und 3 $\mu$m haben.

**6.** Pharmazeutische Formulierung nach Anspruch 5, wobei mindestens 50% der Wirkstoffpartikel einen geometrischen Durchmesser zwischen 1 $\mu$m und 3 $\mu$m haben.

**7.** Pharmazeutische Formulierung nach Anspruch 1, wobei die Lipidmatrix ein Phospholipid umfasst.

**8.** Pharmazeutische Formulierung nach Anspruch 7, wobei das Phospholipid eine Phasenübergangstemperatur von der Gelphase zur Flüssigphase höher als etwa 40°C hat.

**9.** Pharmazeutische Formulierung nach Anspruch 7, wobei das Phospholipid ein Phosphoglycerid ist.

**10.** Pharmazeutische Formulierung nach Anspruch 7, wobei das Phospholipid ein gesättigtes Phospholipid ist.

**11.** Pharmazeutische Formulierung nach Anspruch 7, wobei die Feststoffteilchen des Weiteren ein polyvalentes Kation einschließen, das wirksam ist, die Phasenübergangstemperatur von der Gelphase zur Flüssigphase des Phospholipids zu erhöhen.

**12.** Pharmazeutische Formulierung nach Anspruch 1, wobei die Feststoffteilchen des Weiteren ein polyvalentes Kation einschließen.

**13.** Pharmazeutische Formulierung nach Anspruch 12, wobei das polyvalente Kation ein divalentes Kation ist.

**14.** Pharmazeutische Formulierung nach Anspruch 13, wobei das divalente Kation Ca$^{2+}$, Mg$^{2+}$ oder Zn$^{2+}$ ist.

**15.** Pharmazeutische Formulierung nach Anspruch 1, wobei der Wirkstoff ein antimykotisches Agens ist.

**16.** Pharmazeutische Formulierung nach Anspruch 15, wobei das antimykotische Agens eines oder mehrere von Natamycin, Amphotericin B, Voriconazol und Miconazol umfasst.

**17.** Pharmazeutische Formulierung nach Anspruch 16, wobei das antimykotische Agens Amphotericin B ist.

## Revendications

**1.** Formulation pharmaceutique pour l'administration pulmonaire, comprenant une pluralité de matières particulaires ayant un diamètre médian massique inférieur à 20 $\mu$m, dans laquelle chaque matière particulaire comprend : (a) une matrice lipidique ; et (b) une particule d'un agent actif dans la matrice lipidique, ledit agent actif étant sous forme cristalline et ayant une solubilité dans l'eau inférieure à 1,0 mg/ml, dans laquelle au moins 90% des particules d'agent actif de la formulation ont un diamètre géométrique inférieur à 3 $\mu$m.

**2.** Formulation pharmaceutique selon la revendication 1, dans laquelle la pluralité de matières particulaires a un diamètre médian massique inférieur à 10 $\mu$m.

**3.** Formulation pharmaceutique selon la revendication 2, dans laquelle la pluralité de matières particulaires a un diamètre médian massique inférieur à 5 $\mu$m.

**4.** Formulation pharmaceutique selon la revendication 1, dans laquelle au moins au moins 95% des particules d'agent actif ont un diamètre géométrique inférieur à 3 $\mu$m.

**5.** Formulation pharmaceutique selon la revendication 4, dans laquelle au moins 50% des particules d'agent actif ont un diamètre géométrique compris entre 0,5 $\mu$m et 3 $\mu$m.

6. Formulation pharmaceutique selon la revendication 5, dans laquelle au moins 50% des particules d'agent actif ont un diamètre géométrique compris entre 1 $\mu$m et 3 $\mu$m.

7. Formulation pharmaceutique selon la revendication 1, dans laquelle la matrice lipidique comprend un phospholipide.

8. Formulation pharmaceutique selon la revendication 7, dans laquelle le phospholipide a une température de transition du gel à la phase liquide supérieure à environ 40°C.

9. Formulation pharmaceutique selon la revendication 7, dans laquelle le phospholipide est un phosphoglycéride.

10. Formulation pharmaceutique selon la revendication 7, dans laquelle le phospholipide est un phospholipide saturé.

11. Formulation pharmaceutique selon la revendication 7, dans laquelle les matières particulaires comportent en outre un cation polyvalent efficace pour augmenter la température de transition du gel au liquide du phospholipide.

12. Formulation pharmaceutique selon la revendication 1, dans laquelle les matières particulaires comportent en outre un cation polyvalent.

13. Formulation pharmaceutique selon la revendication 12, dans laquelle le cation polyvalent est un cation divalent.

14. Formulation pharmaceutique selon la revendication 13, dans laquelle le cation divalent est $Ca^{2+}$, $Mg^{2+}$ ou $Zn^{2+}$.

15. Formulation pharmaceutique selon la revendication 1, dans laquelle l'agent actif est un agent antimycosique.

16. Formulation pharmaceutique selon la revendication 15, dans laquelle l'agent antimycosique comprend l'un parmi la natamycine, l'amphotéricine B, le voriconazole et le miconazole, ou plusieurs d'entre eux.

17. Formulation pharmaceutique selon la revendication 16, dans laquelle l'agent antimycosique est l'amphotéricine B.

Characteristic Time for Ostwald Ripening

Solubility of <0.01 mg/ml preferred

# FIG. 1

**FIG. 2**

**FIG. 3**

FIG. 4

**FIG. 5**

FIG. 6

FIG. 7A

FIG. 7B

FIG. 7C

FIG. 7D

FIG. 7E

## REFERENCES CITED IN THE DESCRIPTION

*This list of references cited by the applicant is for the reader's convenience only. It does not form part of the European patent document. Even though great care has been taken in compiling the references, errors or omissions cannot be excluded and the EPO disclaims all liability in this regard.*

**Patent documents cited in the description**

- WO 9501221 A
- WO 9600610 A
- WO 9836825 A
- US 5858410 A
- WO 9916419 A
- WO 9916420 PCT
- WO 9916422 PCT
- WO 0185136 PCT
- WO 0185137 PCT
- US 5874064 A
- US 5855913 A
- US 5985309 A
- US 6503480 B
- US 6372258 B
- US 6358530 B
- US 6077543 A
- US 6051256 A
- US 6001336 A
- US 5985248 A
- US 5976574 A
- WO 0185136 A
- WO 0185137 A
- WO 9531479 A
- WO 9632096 A
- WO 9632149 A
- WO 9916422 A
- WO 9916420 A
- WO 9916421 A
- WO 9524183 A
- US 88831101 A
- WO 0283220 A
- US 6546929 B
- US 61644803 A
- US 4247066 A
- US 4846876 A
- US 6357490 B
- WO 0007572 A
- US 4069819 A
- US 4995385 A
- US 3991761 A
- US 4338931 A
- US 5619985 A
- US 5458135 A
- US 5785049 A
- US 6257233 B
- WO 0072904 A
- US 4114615 A

**Non-patent literature cited in the description**

- **FOX TG.** *Bull Am Phys Soc,* 1956, vol. 1, 123